# EUROPÄISCHE PATENTSCHRIFT

(11) **EP 1 016 434 B1**
(45) Veröffentlichungstag und Bekanntmachung des Hinweises auf die Patenterteilung: **02.08.2006**
(21) Anmeldenummer: 99250442.3
(22) Anmeldetag: 17.12.1999
(51) Int. Cl.: A61N 1/368, A61N 1/39

(54) **Herztherapiegerät**
Cardiac pacemaker
Stimulateur cardiaque

(30) Priorität: 28.12.1998 US 220755
(43) Veröffentlichungstag der Anmeldung: 05.07.2000
(73) Patentinhaber: BIOTRONIK GmbH & Co. KG, 12359 Berlin (DE)
(72) Erfinder: Nigam, Indra B., Lake Oswego, Oregon 97035 (US)
(74) Vertreter: Lindner-Vogt, Karin L.

(56) Entgegenhaltungen:
- EP-A- 0 360 412
- EP-A- 0 538 996
- EP-A- 0 750 921
- US-A- 5 183 040
- US-A- 5 836 971

## Beschreibung

Die Erfindung betrifft ein Herztherapiegerät gemäß dem Oberbegriff des Anspruchs 1.

Herzrhythmusstörungen sind in einer außerordentlichen Vielfalt von Erscheinungsformen bekannt, und ein wesentlicher Teil des in den letzten Jahren betriebenen Entwicklungsaufwandes auf dem Gebiet der implantierbaren Herzschrittmacher und Defibrillatoren ist der zuverlässigen Erkennung qualitativ verschiedener Arrhythmien mit unterschiedlichem Therapiebedarf und der Zuordnung der unter den aktuellen Umständen adäquaten Therapie gewidmet.

Die wichtigsten Kriterien für die Unterscheidung verschiedener Arrhythmiezustände sind dabei nach wie vor Ratenkriterien, d.h. der Betrag und ggfs. zusätzlich die zeitliche Entwicklung (Stabilität, Monotonie etc.) der Frequenz der ventrikulären bzw. atrialen Herzaktionen. Bei der Ableitung einer angemessenen Therapie in Abhängigkeit von den Ratenkriterien werden zunehmend zusätzliche Daten über den Patientenzustand und/oder die Vorgeschichte mit in Betracht gezogen.

So werden in der EP 0 580 128 A2 für die Schrittmachertherapie pathologischer Tachykardien die Ratenkriterien in Abhängigkeit von einem die aktuelle körperliche Aktivität des Patienten kennzeichnenden, mittels eines Aktivitätsfühlers gewonnenen, Statussignal modifiziert. Der entsprechende Algorithmus wird bei Erfassung primärer atrialer Fibrillation außer Kraft gesetzt.

Beim kombinierten Kardioverter/Defibrillator (ICD = Implantable Cardioverter/-Defibrillator) und Schrittmacher gemäß EP 0 360 412 A1 wird (in Fortführung eines Klassifizierungsmodells mit starren Bereichsgrenzen) das Herzratenkontinuum in mehrere Bereiche mit variablen Grenzen unterteilt, und in den Grenzbereichen wird die aktuell anzuwendende Therapie aus einem bereichsweise vorbestimmten Vorrat von Therapien unter Berücksichtigung der Vorgeschichte - speziell der vorangegangenen zeitlichen Entwicklung der Rate - ausgewählt.

In der EP 0 538 996 A1 wird vorgeschlagen, für die erstmalige Erfassung einer Tachyarrhythmie ein anderes Ratenkriterium als für eine Bestätigungs-Erfassung zu einem späteren Zeitpunkt anzuwenden, um den physiologischen Erkenntnissen Rechnung zu tragen, daß (1) grundsätzlich behandlungsbedürftige Tachyarrhythmien relativ häufig ohne Stimulationseingriff spontan enden, jedoch (2) längerwährende Tachykardien zunehmend dringend therapiebedürftig werden.

In der gattungsbildenden US 5 836 971 wird für einen ICD eine dynamische Zuordnung verschiedener Ratenbereiche zu verschiedenen Therapien, die sich im Betrag der angewandten Energie unterscheiden, in Abhängigkeit davon vorgeschlagen, ob und mit welcher Energie bereits ein Therapieversuch unternommen wurde. Damit soll die Durchsetzung des Prinzips der Anwendung steigender Energiebeträge bei aufeinanderfolgenden Therapieversuchen auch dann gesichert werden, wenn sich nach einem Schock eine niedrigere Rate als vorher eingestellt hat, ohne daß das Herz zum normalen Sinusrhythmus zurückgekehrt wäre.

EP0750921 A2 zeigt ein elektromedizinisches Implantat zum Herzstimulation mit einem Steursystem, das zum Vermeidung von Fehlsteuerungen einen doppelten Speichrer für die einzelnen Steurparameter der anstehenden Therapie enthält.

Der Erfindung liegt die Aufgabe zugrunde, ein verbessertes Herztherapiegerät der gattungsgemäßen Art anzugeben, das eine schonendere Therapie von Tachyarrhythmien ermöglicht.

Die Aufgabe wird durch ein Herztherapiegerät mit den im Anspruch 1 angegebenen Merkmalen gelöst.

Die Erfindung schließt die Erkenntnis ein, daß Änderungen einer den Herzzustand kennzeichnenden Größe, speziell Herzratenänderungen, nach einem Therapieversuch für die Festlegung des weiteren Therapieverlaufes auch dann differenziert bewertet werden sollten, wenn der Therapieversuch nicht zur Wiederherstellung des Normalzustandes geführt hat. Bei Erfassung bestimmter Änderungen kann insbesondere eine Abkehr vom Prinzip der Elektrotherapie mit ansteigender Energie bzw. eine Aussetzung weiterer Therapieversuche angemessen sein.

Bei einem ICD bedeutet das etwa, daß bei Erfassung einer Ratenabsenkung nach einem Defibrillationsschock auch dann eine Aussetzung weiterer Therapieversuche erfolgt, wenn die Rate noch in einem Tachykardiebereich liegt, dem eine Therapie zugeordnet ist.

Zur Erfindung gehört das Vorsehen von Mitteln zur Erfassung eines den Zustand des Herz-/Kreislaufsystems kennzeichnenden physiologischen Parameters, zur Speicherung einer Mehrzahl von Vergleichswerten des physiologischen Parameters und zum Vergleich des erfaßten Wertes mit den gespeicherten Werten zur Ausgabe eines Klassifizierungsignals. Sie schließt weiter das Vorhandensein von Mitteln zur Speicherung einer Mehrzahl potentieller Therapien, von Mitteln zur wahlweisen Ausführung einer der potentiellen Therapien, von Mitteln zur Registrierung einer ausgeführten Therapie und von Mitteln zur Auswahl einer der gespeicherten Therapien in Abhängigkeit von dem Klassifizierungssignal ein, wobei speziell Mittel zur Verzögerung der Ausführung oder zur Inhibierung der dem konkret vorliegenden Klassifizierungssignal zugeordneten Therapie - oder auch jedweder Therapie - in Abhängigkeit von dem Klassifizierungssignal und der vorher ausgeführten Therapie (genauer gesagt: eines Therapieversuches) vorgesehen sind.

Angesichts der besonderen Bedeutung der Ratenkriterien für Herztherapiegeräte ist im Rahmen der Erfindung die Erfassung mindestens der aktuellen Herzrate - ggfs. zusätzlich die Bestimmung von deren zeitliche Entwicklung kennzeichnenden Kriterien - vorgesehen, so daß das Klassifizierungssignal die Zuordnung der Herzaktivität zu einem vorbestimmten Ratenbereich kennzeichnet.

Zur Ausführung der Erfindung sind mindestens zwei Vergleichswerte der Herzrate fest vorprogrammiert, womit jedes von mindestens drei Klassifizierungssignalen die Zuordnung der aktuellen Herzrate zu jeweils einem invariant vorbestimmter Ratenbereiche kennzeichnet, von denen mindestens einer als Hysteresebereich in Abhängigkeit von der Therapie-Vorgeschichte definiert ist. Bevorzugt sind aber drei oder vier Vergleichswerte vorprogrammiert, so daß jedes von vier oder fünf Klassifizierungssignalen die Zuordnung der aktuellen Herzrate zu jeweils einem Ratenbereich kennzeichnet, von denen zwei als oberer bzw. unterer Hysteresebereich definiert sind.

Ein implantierbarer Kardioverter/Defibrillator (ICD) oder kombinierter Schrittmacher/-Defibrillator als spezielle Ausführungsform des Therapiegerätes umfaßt einen Schockimpulsgenerator zur Kardioversion bzw. Defibrillation des Herzens und ist zur Verzögerung oder Inhibierung weiterer Schockimpulse nach einem vorausgegangenen Impuls bei Empfang eines ersten Klassifizierungssignals ausgebildet, das eine Herzrate mit erhöhtem Sinusrhythmus anzeigt. (Bei einem kombinierten Schrittmacher/Defibrillator könnte in diesem Falle auch eine Antitachykardie-Stimulierung aktiviert werden, dies ist aber u.U. physiologisch nicht sinnvoll.) Er ist im übrigen zur Auslösung weiterer Schockimpulse mit im wesentlichen gleichem Energiepegel nach dem ersten Impuls bei Empfang eines zweiten Klassifizierungssignals ausgebildet, welches speziell eine Herzrate im Tachyarrhythmiebereich - oberhalb des Bereiches erhöhter Sinusrate, aber unterhalb der Fibrillationsgrenze - anzeigt.

Vorteilhafte Weiterbildungen der Erfindung sind im übrigen in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: als bevorzugtes Ausführungsbeispiel einer erfindungsgemäßen Vorrichtung ein Funktions-Blockschaltbild eines kombinierten Defibrillators/Herzschrittmachers und
- Figur 2: eine grafische Darstellung zur Erläuterung der Arbeitsweise der Vorrichtung nach Fig. 1.

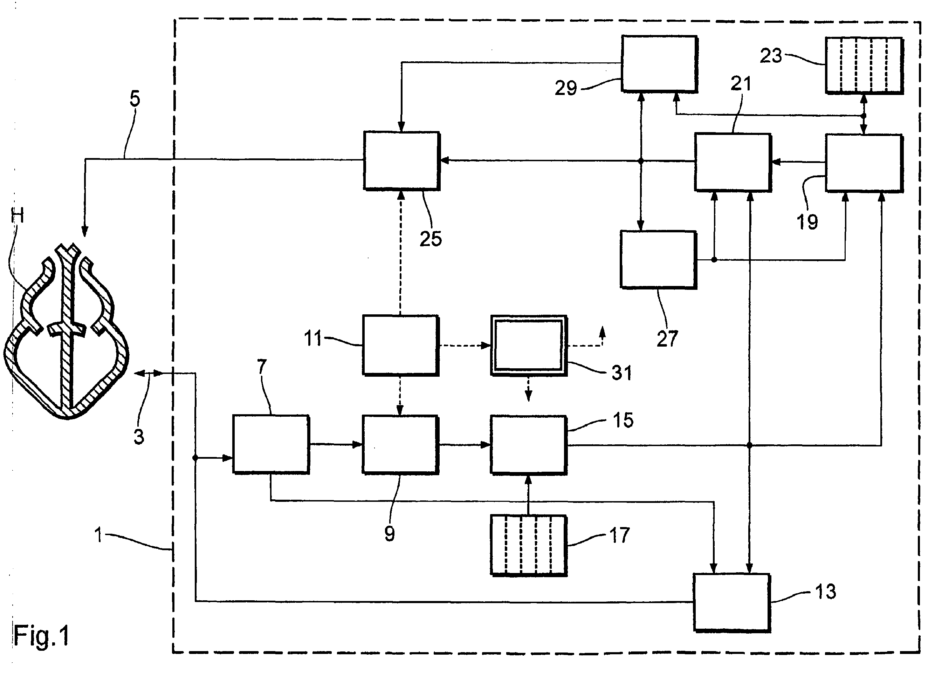

Fig. 1 zeigt in stark vereinfachter Darstellung, die auf die im Zusammenhang mit der Erfindung wesentlichen Komponenten beschränkt ist, als Funktions-Blockschaltbild einen implantierbaren Defibrillator/Herzschrittmacher 1, der über eine Abfühl- und Stimulationselektrodenleitung 3 und eine Defibrillationselektrodenleitung 5 mit dem Herzen H eines Patienten verbunden ist.

Mit der Abfühl- und Stimulationselektrodenleitung 3 ist eingangsseitig eine Herzsignal-Eingangsstufe 7 verbunden, die ausgangsseitig zum einen mit einer Ratenbestimmungsstufe 9, die zudem Zeitsignale von einem Zeitgeber 11 empfängt, und zum anderen mit einem Signaleingang einer Schrittmachereinheit 13 verbunden ist. Die Ratenbestimmungsstufe 9 ist ausgangsseitig mit einem Eingang eines mehrstufigen Ratenkomparators 15 verbunden, der über einen zweiten Eingang mit einem Raten-Bereichsgrenzenspeicher 17 in Verbindung steht.

Der Ausgang des Ratenkomparators 15 ist mit einem Steuereingang der Schrittmachereinheit 13 und weiterhin mit jeweils einem Eingang einer SchocktherapieAuswahleinheit 19 sowie einer dieser nachgeordneten Therapie-Korrekturstufe 21 verbunden. Die Schocktherapie-Auswahleinheit 19 steht zudem mit einem Energiestufenspeicher 23 und ausgangsseitig mit einem weiteren Eingang der Therapie-Korrekturstufe 21 in Verbindung. Diese schließlich ist ausgangsseitig mit einem Schockimpulserzeuger 25, der eigentlichen Defibrillatorstufe, und zudem über eine Signalleitung mit einer Therapie-Registrierstufe 27 verbunden. Die Defibrillatorstufe 25 ist über die Defibrillationsleitung 5 an das Herz H gekoppelt und wird durch eine Energiespeicher- und Aufladestufe 29 gespeist, die ihrerseits eingangsseitig mit dem Energiestufenspeicher 23 sowie dem Ausgang der Therapie-Korrekturstufe 21 verbunden ist.

Der Gesamtbetrieb der Anordnung wird durch eine Mikroprozessorsteuerung 31 gesteuert. Die Vorrichtung arbeitet grundsätzlich wie folgt:

Aus am Herzen abgefühlten intrakardialen Signalen und den intern bereitgestellten Zeitsignalen wird - nach der üblichen Aufbereitung in der Eingangsstufe 7 - die für die Steuerung relevante Herzrate, d.h. bevorzugt die ventrikuläre, ggfs. aber auch die atriale Rate, ermittelt und im Ratenkomparator 15 einem Vergleich mit vorprogrammierten, im Raten-Bereichsgrenzenspeicher 17 gespeicherten Ratenbereichsgrenzen unterzogen. Auf diese Weise wird festgestellt, in welchem diagnostisch relevanten Ratenbereich die Herzaktivität aktuell liegt; Einzelheiten dazu siehe weiter unten.

Im Ergebnis des Vergleiches gibt der Ratenkomparator ein Klassifizierungssignal aus, das als entscheidendes Steuersignal parallel zur Schrittmachereinheit 13, zur Schocktherapie-Auswahleinheit 19 und zur Therapie-Korrekturstufe 21 gelangt. Je nach Art des Klassifizierungssignals wird - wie üblich, unter zusätzlicher Steuerung durch das Ausgangssignal der Eingangsstufe 7 - entweder ein Stimulationsbetrieb der Schrittmachereinheit 13 oder ein Defibrillatorbetrieb gesteuert, oder die Vorrichtung übt keine therapeutische Wirkung aus.

Was den Defibrillatorteil angeht, so wird gemäß den weiter unten genauer erläuterten Kriterien durch die Schocktherapie-Auswahleinheit 19 im Ansprechen auf das Klassifizierungssignal ggfs. einer von mehreren vorprogrammierten, im Speicher 23 gespeicherten Schocktherapiemodi ausgewählt und ein entsprechendes Steuersignal sowohl an die Therapie-Korrekturstufe 21 als auch an die Energiespeicher- und Aufladestufe 29 übermittelt, wo daraufhin in üblicher Weise die benötigte Schockenergie bereitgestellt wird.

In der Therapie-Korrekturstufe 21 erfolgt anhand des Klassifizierungssignals vom Ratenkomparator 15 und eines die vorangegangene(n) Therapie(n) kennzeichnenden Ausgangssignals der Therapie-Registrierstufe 27 eine Nachverarbeitung des durch die Schocktherapie-Auswahleinheit 19 ausgegebenen Steuersignals, durch die die Therapie-Vorgeschichte berücksichtigt wird. Im Ergebnis dieser Nachverarbeitung wird in Abhängigkeit vom konkreten Klassifikationssignal speziell, sofern der Modus "Defibrillationsschock" eingestellt war, dieser entweder beibehalten oder jede Schocktherapie inhibiert. War im vorhergehenden Therapieschritt ein anderer Modus gewählt, kann das Ergebnis der Nachverarbeitung anderen Randbedingungen unterliegen, oder die Therapie-Korrekturstufe 21 wird überhaupt nur durch die Auswahl dieses Modus für eine nachfolgende Überprüfungs- bzw. Therapiephase aktiviert. Durch das Ausgangssignal der Therapie-Korrekturstufe 21 schließlich wird - nach Beendigung der Aufladung des (nicht gesondert dargestellten) Energiespeichers - der Schockimpulserzeuger 25 aktiviert oder aber inhibiert und ein Schockimpuls an das Herz abgegeben oder unterdrückt.

Durch die Mikroprozessorsteuerung 31 wird zu einem vorbestimmten späteren Zeitpunkt ein erneuter Auswertungs- und Entscheidungszyklus gesteuert, der wiederum auf die oben beschriebene Weise ablaufen oder aber modifiziert sein kann - insbesondere dahingehend, daß nach Verstreichen eines längeren Zeitraumes eine andere Betriebsweise der Therapie-Korrekturstufe 21 gewählt oder diese deaktiviert wird, so daß dann die Therapieauswahl wieder unabhängig von der Therapie-Vorgeschichte, insbesondere der zwischenzeitlichen Abgabe eines Defibrillationsschocks, wird.

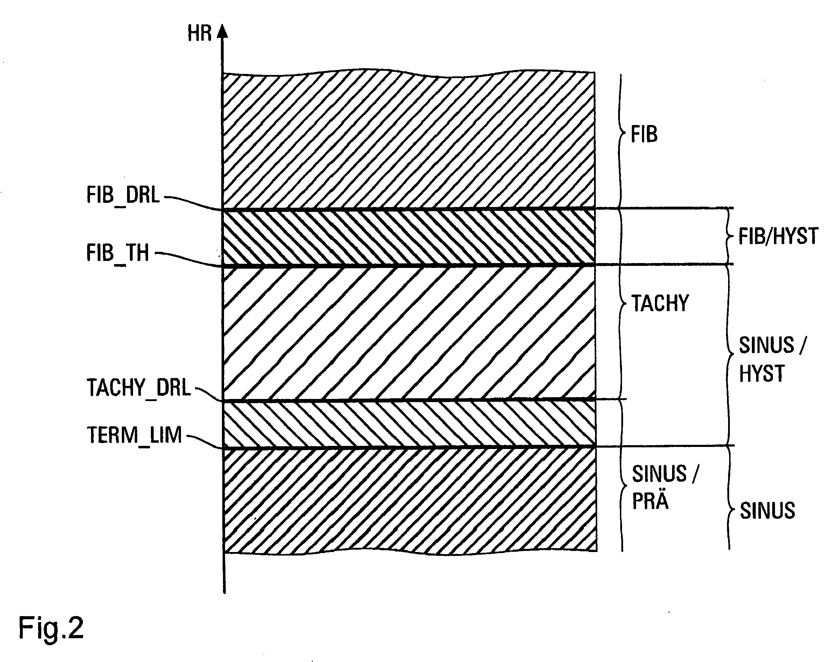

Fig. 2 ist eine schematische Darstellung des Herzratenkontinuums (mit in Richtung der y-Achse zunehmenden Ratenwerten HR) mit den Ratenbereichsgrenzen FIB_DRL (Ratengrenze zwischen Tachykardie- und Fibrillationsbereich), FIB_TH (untere Ratengrenze für Post-Schock-Defibrillationstherapie), TACHY_DRL (Ratengrenze zwischen Sinusraten- und Tachykardiebereich), TERM_LIM (oberer Ratengrenzwert für eine als erfolgreich anzusehende Defibrillation. Die Ratenbereichsgrenzen definieren, wie aus der Figur ersichtlich ist, die Ratenbereiche FIB, FIB/HYST (nur definiert nach einem Schockimpuls), TACHY (nur definiert vor einem Schockimpuls), SINUS/HYST (nur definiert nach einem Schockimpuls), SINUS/PRÄ (nur definiert vor einem Schockimpuls) und SINUS (nur definiert nach einem Schockimpuls), wobei zu beachten ist, daß die Hysteresebereiche FIB/HYST und SINUS/HYST auf unterschiedliche Weise definiert sind:

Der obere Hysteresebereich FIB/HYST umfaßt lediglich einen (oberen) Teilbereich des Bereiches TACHY - der für die Ratenauswertung zur Festlegung der weiteren Therapie nach einem Defibrillationsschock quasi aufgeteilt wird - , während der untere Hysteresebereich SINUS/HYST einen (oberen) Teilbereich des vor einem Schock definierten Bereiches SINUS/PRÄ und den gesamten restlichen TACHYBereich umfaßt.

In einer zur erleichterten Auswertung vereinfachten Ausführung fallen die Grenzen TACHY_DRL und TERM_LIM und damit die Bereiche SINUS/PRÄ und SINUS zusammen, so daß der untere Hysteresebereich SINUS/HYST allein dem unteren Tachykardie-Teilbereich entspricht.

Bei dem gezeigten Ratendiagramm wird bei einer Rate im Bereich FIB eine Defibrillationstherapie ausgelöst; die Unterscheidung zwischen den Bereichen SINUS/PRÄ und TACHY ist nur für einen kombinierten Antitachykardieschrittmacher/Defibrillator relevant, bei dem eine Rate im Bereich TACHY - vor einem Schockimpuls - eine Antitachykardiestimulation, etwa eine (an sich bekannte) Overdrive- oder orthorhythmische Stimulation, auslöst.

Sinkt nach einem Defibrillationsschock die Rate in den Bereich SINUS, ist die Defibrillationstherapie erfolgreich abgeschlossen; üblicherweise wird dann ein normaler Überwachungsbetrieb wiederaufgenommen.

Sinkt sie nur in den Bereich SINUS/HYST, so ist zwar eine weitere spezielle Beobachtung angezeigt, es wird aber die Ausgabe weiterer Schockimpulse ausgesetzt, da angenommen wird, daß das Herz aus dem Fibrillationszustand in physiologisch günstiger Weise in einen Zustand von Sinusaktivität mit erhöhter Frequenz übergegangen ist. (Es ist übrigens nicht sinnvoll, bei einer Rate in diesem Bereich eine völlige Beendigung der Therapie festzulegen, weil dann anschließend - im normalen Prä-Schock-Detektionsmodus - eine Tachykardie erfaßt werden und bei einem kombinierten Antitachykardieschrittmacher/Defibrillator in physiologisch unzweckmäßiger Weise eine Antitachykardiestimulation initiiert würde.)

Sinkt die Rate hingegen nur geringfügig in den Bereich FIB/HYST, so wird - ebenso wie natürlich bei ihrem Verbleiben im Bereich FIB - mit der Schockimpulstherapie normal fortgefahren, sofern hierfür keine anderweitige Limitierung wirkt. Die Erfindung beschränkt sich in ihrer Ausführung nicht auf das vorstehend angegebene bevorzugte Ausführungsbeispiel. Vielmehr ist eine Anzahl von Varianten möglich, welche von der dargestellten Lösung auch in anders gearteten Ausführungen Gebrauch machen.

So kann das Herztherapiegerät - bei grundsätzlich gleichartiger Auswertung der Herzrate - auch als Medikamentendosiergerät oder als Nervenstimulator - insbesondere zur Vagusstimulation - bzw. als kombinierter Nervenstimultor/Herzschrittmacher zur medikamentösen oder neurostimulativen Fibrillationstherapie ausgeführt sein.

Es können Mittel zur Unterscheidung einer größeren Anzahl von Herzratenbereichen vorgesehen sein, und die Klassifizierung der Herzaktivität muß nicht oder nicht primär aufgrund des Absolutwertes der Herzrate bzw. von deren zeitlicher Entwicklung erfolgen, sondern kann insbesondere eine Auswertung der intrakardial erfaßten Herzsignal(IECG)-Morphologie einschließen. Die Signalmorphologie ist nämlich unter bestimmten Umständen und bei geeigneter Signalverarbeitung ein brauchbares Kriterium beispielsweise für die Unterscheidung zwischen SinusAktivität einerseits und (eine Fortsetzung der Schocktherapie erfordernden) Tachy-arrhythmien andererseits.

## Patentansprüche

1. Herztherapiegerät (1) mit
Erfassungsmitteln (3, 7, 9) zur Erfassung eines den Zustand des Herz-/Kreislaufsystems kennzeichnenden physiologischen Parameters (HR),
Vergleichswert-Speichermitteln (17) zur Speicherung einer Mehrzahl von Vergleichswerten des physiologischen Parameters, wobei die Erfassungsmittel (3, 5, 9) zur Erfassung mindestens der Herzrate (HR) und die Vergleichswert-Speichermittel (17) zur Speicherung einer Mehrzahl von Vergleichswerten der Herzrate (HR) ausgebildet sind derart, daß das Klassifizierungssignal die Zuordnung der aktuellen Herzrate zu einem vorbestimmten Ratenbereich (SINUS/PRÄ, SINUS, SINUS/HYST, TACHY, FIB/HYST, FIB) kennzeichnet
eingangsseitig mit den Erfassungsmitteln und den Vergleichswert-Speichermitteln verbundenen Vergleichermitteln (15) zum Vergleich des erfaßten Wertes mit den gespeicherten Werten und zur Ausgabe eines Klassifizierungsignals im Ergebnis des Vergleiches, Therapie-Speichermitteln (23) zur Speicherung einer Mehrzahl potentieller Therapien für ein Herz (H),
Therapiemitteln (25) zur wahlweisen Ausführung einer der potentiellen Therapien,
Therapie-Registriermitteln (27) zur Registrierung einer ausgeführten Therapie,
eingangsseitig mit dem Ausgang der Vergleichermittel (15) und den Therapie-Registriermitteln (27) verbundenen Therapieauswahlmitteln (19) zur Auswahl einer der gespeicherten Therapien und zur Aktivierung der Therapiemittel (25) in Abhängigkeit von dem Ktassifizierungssignat und dem Speicherinhalt der Therapie-Registriermittel (27), **gekennzeichnet durch** den Therapie-Auswahlmitteln (19) zugeordnete Therapie-Korrekturmittel (21) zur Verzögerung oder Inhibierung der Ausführung einer dem Klassifizierungssignal zugeordneten Therapie oder jedweder Therapie in Abhängigkeit von dem Klassifizierungssignal und dem Speicherinhalt der Therapie-Registriermittel (27), wobei mindestens zwei Vergleichswerte (TERM_LIM, TACHY_DRL, FIB_TH, FIB) der Herzrate fest vorprogrammiert sind derart, daß jedes von mindestens drei Klassifizierungssignalen die Zuordnung der aktuellen Herzrate zu jeweils einem invariant vorbestimmten Ratenbereich (SINUS/PRÄ, SINUS, SINUS/HYST, TACHY, FIB/HYST, FIB) kennzeichnet, wobei mindestens ein Ratenbereich (SINUS/HYST, FIB/HYST) nach einem Therapieversuch als Hysteresebereich definiert ist, in dem die Therapie-Korrekturmittel (21) in Abhängigkeit von der Therapie-Vorgeschichte wirken.

2. Herztherapiegerät nach Anspruch 1, **dadurch gekennzeichnet, daß** die Therapiemittel einen Schockimpulsgenerator (25) zur Erzeugung von Schockimpulsen zur Kardioversion bzw. Defibrillation des Herzens aufweisen und die Therapie-Korrekturmittel (21) zur Verzögerung oder Inhibierung weiterer Schockimpulse nach einem Schockimpuls bei Empfang eines ersten Klassifizierungssignals ausgebildet sind.

3. Herztherapiegerät nach Anspruch 1, **dadurch gekennzeichnet, daß** die Therapiemittel einen Schockimpulsgenerator (25) zur Erzeugung von Schockimpulsen zur Kardioversion bzw. Defibrillation des Herzens aufweisen und die Therapie-Korrekturmittel (21) zur Auslösung weiterer Schockimpulse mit im wesentlichen gleichem Energiepegel nach einem Schockimpuls bei Empfang eines zweiten Klassifizierungssignals ausgebildet sind.

4. Herztherapiegerät nach Anspruch 2, **dadurch gekennzeichnet, daß**das erste Klassifizierungssignal eine Herzrate im Bereich (SINUS/HYST) erhöhten Sinusrhythmus unterhalb einer vorbestimmten Tachykardie-Ratengrenze (FIB_TH) kennzeichnet.

5. Herztherapiegerät nach Anspruch 3, **dadurch gekennzeichnet, daß** das zweite Klassifizierungssignal eine Herzrate im einem Tachykardiebereich (FIB/HYST) oberhalb einer Tachykardie-Ratengrenze (FIB_TH) kennzeichnet.

6. Herztherapiegerät nach Anspruch 5, **dadurch gekennzeichnet, daß** der durch das zweite Klasifizierungssignal gekennzeichente Tachykardiebereich (FIB/HYST) unterhalb einer Fibrillationsratengrenze (FIB) liegt.

7. Herztherapiegerät nach Anspruch 1, **dadurch gekennzeichnet, daß** vier Vergleichswerte (TERM_ LIM, TACHY_DRL, FIB_TH, FIB) der Herzrate fest vorprogrammiert sind derart, daß jedes von fünf Klassifizierungssignalen die Zuordnung der aktuellen Herzrate zu jeweils einem vorbestimmten Ratenbereich kennzeichnet, wobei zwei Ratenbereiche (SINUS/HYST, FIB/HYST) als Hysteresebereiche definiert sind, in denen die Therapie-Korrekturmittel (21) wirken.

## Claims

1. A cardiac treatment device (1) having
detection means (3, 7, 9) for detecting a physiological parameter (HR) identifying the state of the cardiovascular system,
comparative value storage means (17) for storing multiple comparative values of the physiological parameter, the detection means (3, 5, 9) for detecting at least the heart rate (HR) and the comparative value storage means (17) for storing multiple comparative values of the heart rate (HR) being implemented in such way that the classification signal identifies the assignment of the current heart rate to a predetermined rate range (SINus/PRA, SINUS, SINUS/HYST, TACHY, FIB/HYST, FIB),
comparator means (15), which are connected at the input to the detection means and the comparative value storage means, for comparing the detected value to the stored values and for outputting a classification signal as a result of the comparison,
treatment storage means (23) for storing multiple potential treatments for a heart (H),
treatment means (25) for alternately executing one of the potential treatments,
treatment registration means (27) for registering an executed treatment,
treatment selection means (19), which are connected at the input to the output of the comparator means (15) and the treatment registration means (27), for selecting one of the stored treatments and for activating the treatment means (25) as a function of the classification signal and the memory content of the treatment registration means (27), **characterized by** treatment correction means (21), which are assigned to the treatment selection means (19), for delaying or inhibiting the execution of a treatment assigned to the classification signal or any treatment as a function of the classification signal and the memory contents of the treatment registration means (27), at least two comparative values (TERM_LIM, TACHY_DRL, FIB_TH, FIB) of the heart rate being permanently preprogrammed in such way that each of at least three classification signals identifies the assignment of the current heart rate to an invariant, predetermined rate range (SINUS/PRA, SINUS, SINUS/HYST, TACHY, FIB/HYST, FIB) in each case, at least one rate range (SINUS/HYST, FIB/HYST) being defined as a hysteresis range after a treatment attempt, in which the treatment correction means (21) act as a function of the previous treatment history.

2. The cardiac treatment device according to claim 1, **characterized in that** the treatment means have a shock pulse generator (25) for generating shock pulses for cardioversion and/or defibrillation of the heart and the treatment correction means (21) are implemented to delay or inhibit further shock pulses after a shock pulse upon receiving a first classification signal.

3. The cardiac treatment device according to claim 1, **characterized in that** the treatment means have a shock pulse generator (25) for generating shock pulses for cardioversion and/or defibrillation of the heart and the treatment correction means (21) are implemented to trigger further shock pulses having essentially identical energy level after a shock pulse upon receiving a second classification signal.

4. The cardiac treatment device according to claim 2, **characterized in that** the first classification signal identifies a heart rate in the range (SINUS/HYST) of elevated sinus rhythm below a predetermined tachycardia rate boundary (FIB_TH).

5. The cardiac treatment device according to claim 3, **characterized in that** the second classification signal identifies a heart rate in a tachycardia range (FIB/HYST) above a tachycardia rate boundary (FIB_TH).

6. The cardiac treatment device according to claim 5, **characterized in that** the tachycardia range (FIB/HYST) **characterized by** the second classification signal lies below a fibrillation rate boundary (FIB).

7. The cardiac treatment device according to claim 1, **characterized in that** four comparative values (TERM_LIM, TACHY_DRL, FIB_TH, FIB) of the heart rate are permanently preprogrammed in such way that each of five classification signals identifies the assignment of the current heart rate to a predetermined rate range in each case, two rate ranges (SINUS/HYST, FIB/HYST) being defined as hysteresis ranges, in which the treatment correction means (21) act.

## Revendications

1. Appareil de thérapie cardiaque (1), comprenant
des moyens d'enregistrement (3, 7, 9) pour l'enregistrement d'un paramètre (HR) physiologique caractérisant l'état du système cardiaque/circulatoire,
des moyens de mémorisation de valeurs comparatives (17) pour l'enregistrement d'une pluralité de valeurs comparatives du paramètre physiologique, les moyens d'enregistrement (3, 5, 9) pour l'enregistrement au moins du rythme cardiaque (HR) et des moyens de mémorisation des valeurs de comparaison (17) pour le stockage d'une pluralité de valeurs de comparaison du rythme cardiaque (HR) sont réalisés de telle sorte que le signal de classification caractérise l'attribution du rythme cardiaque actuel à une plage de rythme prédéfinie (SINUS/PRÀ, SINUS, SINUS/HYST, TACHY, FIB/IST, FIB),
des moyens de comparaison (15) reliés côté entrée aux moyens d'enregistrement et aux moyens de stockage de valeurs de comparaison pour la comparaison de la valeur enregistrée avec les valeurs mémorisées et pour la sortie d'un signal de classification en conclusion de la comparaison,
des moyens de stockage de thérapie (23) pour le stockage d'une pluralité de thérapies potentielles pour un coeur (H),
des moyens de thérapie (25) pour l'exécution optionnelle de l'une des thérapies potentielles,
des moyens d'enregistrement de thérapie (27) pour l'enregistrement d'une thérapie réalisée,
des moyens de sélection de thérapie (19) reliés côté entrée à la sortie des moyens du comparateur (15) et aux moyens d'enregistrement de thérapie (27) pour la sélection de l'une des thérapies mémorisées et pour l'activation des moyens de thérapie (25) en fonction du signal de classification et du contenu de mémoire des moyens d'enregistrement de thérapie (27), **caractérisé par** les moyens de correction de thérapie (21) attribués aux moyens de sélection de thérapie (19) pour le retardement ou l'inhibition de la réalisation d'une thérapie attribuée au signal de classification ou de toute thérapie en fonction du signal de classification ou du contenu de mémoire des moyens d'enregistrement de thérapie (27),
au moins deux valeurs de comparaison (TERM_LIM, TACHY_DRL, FIB_TH, FIB) du rythme cardiaque étant pré-programmées de façon fixe de telle sorte que chacun de au moins trois signaux de classification caractérise l'attribution du rythme cardiaque actuel à respectivement une plage de rythme prédéfinie de façon invariable (SINUS/PRÀ, SINUS, SINUS/HYST, TACHY, FIB/HYST, FIB), au moins une plage de rythmes (SINUS/HYST, FIB/HYST) étant définie après un essai de thérapie comme plage d'hystérésis, dans laquelle les moyens de correction de thérapie (21) agissent en fonction de l'anamnèse de thérapie.

2. Appareil de thérapie cardiaque selon la revendication 1, **caractérisé en ce que** les moyens de thérapie présentent un générateur d'impulsion de choc (25) pour la génération d'impulsions de choc pour la version cardio et la défibrillation du coeur et les moyens de correction de thérapie (21) sont conçus pour le retardement ou l'émission d'autres impulsions de choc après une impulsion de choc en cas de réception d'un premier signal de classification.

3. Appareil de thérapie cardiaque selon la revendication 1, **caractérisé en ce que** les moyens de thérapie présentent un générateur d'impulsion de choc (25) pour la génération d'impulsions de choc pour la version cardio et la défibrillation du coeur et les moyens de correction de thérapie (21) sont réalisés pour le déclenchement d'autres impulsions de choc avec un niveau d'énergie sensiblement identique après une impulsion de choc en cas de réception d'un second signal de classification.

4. Appareil de thérapie cardiaque selon la revendication 2, **caractérisé en ce que** le premier signal de classification caractérise un rythme cardiaque dans la plage (SINUS/HYST) de rythme sinusal élevé au-dessous d'une limite de rythme de tachycardie (FIB TH) prédéfinie.

5. Appareil de thérapie cardiaque selon la revendication 3, **caractérisé en ce que** le second signal de classification caractérise un rythme cardiaque dans une plage de tachycardie (FIB_TH) au-dessus d'une limite de rythme de tachycardie (FIB_TH).

6. Appareil de thérapie cardiaque selon la revendication 5, **caractérisé en ce que** la plage de tachycardie (FIB/HYST) **caractérisée par** le second signal de classification se situe au-dessous d'une limite de rythme de fibrillation (FIB).

7. Appareil de thérapie cardiaque selon la revendication 1, **caractérisé en ce que** quatre valeurs de comparaison (TERM_LIM, TACHY_DRL, FIB_TH, FIB) du rythme cardiaque sont pré-programmées de façon fixe de telle sorte que chacun de cinq signaux de classification caractérise l'attribution du rythme cardiaque actuel à respectivement une plage de rythme prédéfinie, deux plages de rythme SINUS/HYST, FIB/HYST) étant définies comme des plages d'hystérésis dans lesquelles les moyens de correction de thérapie (21) agissent.
